(19) Europäisches Patentamt
European Patent Office
Office européen des brevets

(11) **EP 3 888 547 A1**

(12) **EUROPEAN PATENT APPLICATION**

(43) Date of publication:
**06.10.2021 Bulletin 2021/40**

(51) Int Cl.:
***A61B 5/11*** *(2006.01)*

(21) Application number: **20167671.5**

(22) Date of filing: **02.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **Huijbregts, Laurentia Johanna**
  **5656 AE Eindhoven (NL)**
- **Sartor, Francesco**
  **5656 AE Eindhoven (NL)**

(74) Representative: **Philips Intellectual Property & Standards**
**High Tech Campus 5**
**5656 AE Eindhoven (NL)**

(54) **DEVICE, SYSTEM AND METHOD FOR GENERATING INFORMATION ON MUSCULOSKELETAL RECOVERY OF A SUBJECT**

(57) The present invention relates to a device, system and method for generating information on musculoskeletal recovery of a subject. To enable the generation of additional information on recovery of a subject to better avoid over-fatigue, injuries and overuse fractures, the device (10) comprises a sensor input (11) configured to receive a motion parameter and/or motion signal related to motion of the subject performing an activity; a processor (12) configured to determine from the motion parameter and/or motion signal a measure of impact load, determine from the measure of impact load a measure of musculoskeletal damage, and determine from the measure of impact load information on musculoskeletal recovery indicating to which extent the musculoskeletal damage recovers over time; and an output (13) configured to output the determined information on musculoskeletal recovery.

FIG.1

**Description**

FIELD OF THE INVENTION

The present invention relates to a device, system and method for generating information on musculoskeletal recovery of a subject.

BACKGROUND OF THE INVENTION

A major concern for athletes is to improve their performance. This is a feeling shared by top elite athletes as well as recreational athletes. In order to do so, they all need to exercise (i.e. perform an activity) on a regular basis. Each exercise training session is supposed to disturb their physiology and to induce adaptations, which make the muscles, the lungs, the joints, and the circulation fit to perform a given task. Yet, over-exercising and/or under-recovering can have the opposite effect leading to over-training and/or injuries which may result in a reduction in performance.

To prevent under- or overtraining, sports devices have been giving advice on how long the person should recover from the training. At first, this advice is purely based on the training itself, especially on the heart rate zones and the duration in those. Recently, solutions have appeared that also take into account cognitive stress and sleep quality outside these trainings, as cognitive stress and sleep quality impact how fast a person recovers. These solutions are based on heart rate and heart rate variability features and therefore the recovery of the cardiovascular system. Examples of solutions for recovery are the Garmin Body Battery based on Firstbeat algorithms (described e.g. in US 7,192,401) and Polar Recovery Pro.

US 2016/220866 A1 discloses a device that helps a user to plan the proper timing for setting a next training session based on the intensity of a training stimulus of a previous session. The user is shown when there will have been enough recovery time since the last training session to suggest the best time for starting the next training session. The timing recommendations are based on a supercompensation time curve that depends on a user dependent factor that includes at least the training load of the last training session and preferably also depends on the training status of the user. Further parameters like age and gender and variables like behavior affecting recovery, as there are for example tiredness from lack of sleep, dehydration from insufficient drinking, insufficient calories, protein, mineral or vitamin intake, consumption of alcohol and other drugs, can additionally be used to influence the recovery timing calculations.

The existing approaches fail to account for mechanical micro-traumas occurring regularly during training and competitions. These micro-traumas are known to lead to over-fatigue, injuries and overuse fractures.

SUMMARY OF THE INVENTION

It is an object of the present invention to provide a device, system and method for generating additional information on recovery of a subject to better avoid over-fatigue, injuries and overuse fractures.

In a first aspect of the present invention a device for generating information on musculoskeletal recovery of a subject is presented comprising:

- a sensor input configured to receive a motion parameter and/or motion signal related to motion of the subject performing an activity;
- a processor configured to

     determine from the motion parameter and/or motion signal a measure of impact load,
     determine from the measure of impact load a measure of musculoskeletal damage, and
     determine from the measure of impact load information on musculoskeletal recovery indicating to which extent the musculoskeletal damage recovers over time, and

- an output configured to output the determined information on musculoskeletal recovery.

In a further aspect of the present invention a system for generating information on musculoskeletal recovery of a subject is presented comprising:

- a sensor configured to measure a motion parameter and/or motion signal related to motion of the subject;
- a device configured to generate from the motion parameter and/or motion signal information on musculoskeletal recovery of the subject; and
- a user interface to issue the generated information on musculoskeletal recovery.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that the recovery time of muscles, joints, and tendons, which need to recover from trainings as well, generally differs from the cardiovascular recovery time. Thus, it is measured how much the muscles, joints, and tendons, i.e. the subject's musculoskeletal system, have suffered from the training, which information is then used to estimate how long they need to recover. Mechanical micro-traumas occurring regularly during training and competitions and potentially leading to over-fatigue, injuries and overuse fractures are hence taken into account to provide information on musculoskeletal recovery, such as the recovery time, to the subject (or a user, such as a trainer of the subject).

Known solutions generally take into account the cardio-respiratory fatigue, which does not take into account the mechanical traumas induced by repetitive impacts, such as the ones determined by foot-ground contacts per heel strike after double limb unsupported float. The burden on the musculoskeletal system during exercise is not proportional to the burden on the cardiovascular system and consequently its recovery is different, e.g. has a different time constant. The proposed solution determines the biomechanical burden from a measure for impact load and estimates the required recovery, in particular the biomechanical recovery time, of the musculoskeletal system in order to prevent long-term adverse events, such as injuries or stress fractures. In other words, the present invention clearly distinguishes between a cardiovascular load and a musculoskeletal load and separately provides information on recovery in view of the musculoskeletal load of the subject.

There are generally many ways to determine the measure of impact load, depending e.g. on the kind of application, the kind of sensor, etc. In an embodiment the processor is configured to determine the measure of impact load from one or more of the maximum force of a step times the number of steps, the maximum force of a step times the cadence times the duration at this cadence, the speed times the duration of the speed, average magnitude of acceleration times its duration, the peak magnitude of acceleration times its duration, integral of electromyography (EMG) signal over time, maximum torque or force per repetition times the number of repetitions, vertical oscillation times the number of steps, and step length times the number of steps.

In another embodiment the processor is configured to determine the measure of musculoskeletal damage based on a predetermined relationship, in particular a linear relationship, with the determined measure of impact load. Generally, how the measure of musculoskeletal damage is determined depends on which measure is used for the measure of impact load, e.g. how many muscle cells are damaged and need to be repaired by the body. Musculoskeletal damage is defined as ultrastructural damages of the muscle (fibers), connective, skeletal tissue cells, which leads to partial or total loss of key excitation-contraction and of elastic and flexibility functionalities of all those cells and their integral parts, which leads to an inefficient muscle contraction and mechanical shocks dampening capacities. Running-induced musculoskeletal damage may be determined by the morphology of the vertical Ground Reaction Force $GRF_V=$ mb g (tc+ta/tc), where mb is the body mass, g is acceleration of gravity, tc is contact time (of foot with ground) and ta is aerial time (foot not in contact with ground). It is derived that the body segment acceleration (running speed related) and the weight of the person determine the entity of the impact of such segment (e.g. foot, ankle, knee, leg in general), and this in turn determines the physical traumas (mechanical fatigue), damage $\propto GRFv$ + number of GR (ground reactions, or impulses).

In another embodiment the processor is configured to determine the information on musculoskeletal recovery from the measure of impact load information by use of musculoskeletal condition loss at a known moment in time and a time constant for musculoskeletal recovery. One exemplary embodiment determines the degree of musculoskeletal recovery (%) as 100% - C2*EXP(-t/TAU2), wherein C2 is the musculoskeletal condition loss at the end of the training (t=0), is equal to 50%, and TAU2 is a time constant for musculoskeletal recovery, e.g. equal to 2.5 days. One advantage of the disclosed solution is that musculoskeletal recovery is calculated separately, in particular separate from cardiovascular recovery. The entity of $GRF_V$ (derived from a motion sensor) and the number (derived from step count also coming for a motion sensor) may determine the amount of damage to be recovered. Hence, one possible assumption is that $\Sigma(GRF_V)$ (wherein the acceleration derived does not need to be the actual force) will determine the starting level of mechanical fatigue C2. If $\Sigma(GRF_V)$ is large, then C2 is large and vice versa. The morphology of $GRF_V$ also determines the magnitude of TAU2, since for a long run (e.g. 60 min+) the summations of areas under the curve of $GRF_V$ would be larger than for higher speeds but shorter runs. TAU2 for a long run would therefore be greater.

The processor may be configured in an embodiment to determine the information on musculoskeletal recovery based on one of a default formula, a formula with personalized parameters, a formula dependent on the motion of the subject, or a formula having a fluctuating behaviour. Generally, which particular implementation to choose is a matter of simplicity of the device and method versus accuracy of the determined level of musculoskeletal recovery. Using default constant values results in a rather simple implementation, whereas using a personalized formula (dependent on the motion and/or fluctuating) increase the accuracy but may result in a higher complexity of the implementation and a larger need of additional information (such as sleep stages, stress and/or way of running). This additional information may e.g. be obtained from manual input of the user, requiring effort from the user, which might not always be desired.

The information on musculoskeletal recovery may include various pieces of information. In an embodiment the processor may be configured to determine the information on musculoskeletal recovery including one or more of a recovery time indicating the optimal time the subject needs to wait before a new activity should be started, a damage number that decreases over time when there is no new activity, and a recovery number that increases over time when there is no new activity.

The processor may further be configured to determine, in addition to information on musculoskeletal recovery, additional information on cardiovascular recovery and/or on another recovery-related aspect, and the output may be configured to output the determined additional information separate from the information on musculoskeletal recovery or as combined information on recovery determined from the information on musculoskeletal recovery and the additional information, wherein the another recovery-related aspect is one or more of nutrition, fluid level, mental energy, cardiovascular stamina, illness, symptoms, and medication. This provides further information for the subject to improve recovery and avoid over-fatigue, injuries and overuse fractures.

The processor may be configured to determine the measure of musculoskeletal damage from the measure of impact load at the latest activity of the subject and optionally in addition from one or more earlier activities of the subject. This increases the accuracy and reliability of the information on recovery and thus further supports efficient recovery of the subj ect.

The processor may further be configured to take into account, in determining the measure of impact load, the hardness of the surface on which the subject performs the activity and/or a foot strike pattern. This further improves the accuracy and reliability of the information on recovery as well and thus further supports efficient recovery of the subject. For instance, the processor may be configured to determine if the surface is damped from irregularity of the motion parameter or motion signal and/or from sound of steps of the subject performing the activity and/or to distinguish forefoot strike from heel strike by using cadence.

The processor may further be configured to determine the information on musculoskeletal recovery separately for one or more different body parts. This enables an individual recovery and individual training of those body parts.

The disclosed system comprises a sensor, a device as described above and a user interface. The sensor may include one or more of a wearable accelerometer, wearable force sensor, wearable pressure sensor, wearable electromyography sensor, wearable global positioning system sensor, remote camera, radar, speed measurement sensor, and a torque measurement sensor and/or wherein the sensor is an arm- or wrist-worn sensor. The user interface may be configured to allow input of subject-related and/or environment-related information, in particular of one or more of a type of surface, a type of shoes of the subject, body height of the subject, weight of the subject, age of the subject, and existing and/or previous injuries of the subject.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system and a device according to the present invention;
Fig. 2A shows a diagram of the gait cycle phases during running;
Fig. 2B shows a diagram illustrating accelerometer signals for accelerometers placed on different places on the body during running;
Fig. 3 shows a diagram illustrating an example of cardiovascular recovery, recovery from mechanical impact and combined recovery;
Fig. 4 shows a diagram illustrating an embodiment of giving feedback to the subject;
Fig. 5 shows a diagram illustrating another embodiment of giving feedback to the subject;
Fig. 6 shows a diagram illustrating the degree of recovery for the cardiovascular system and the musculoskeletal system over 24 hours;
Fig. 7A shows a map of the subject's body right after training illustrating which body parts are affected by the training and to which extent;
Fig. 7B shows a map of the subject's body twelve hours after training illustrating which body parts are have recovered from training and to which extent; and
Fig. 8 shows a flowchart illustrating an embodiment of a method according to the present invention.

DETAILED DESCRIPTION OF THE EMBODIMENTS

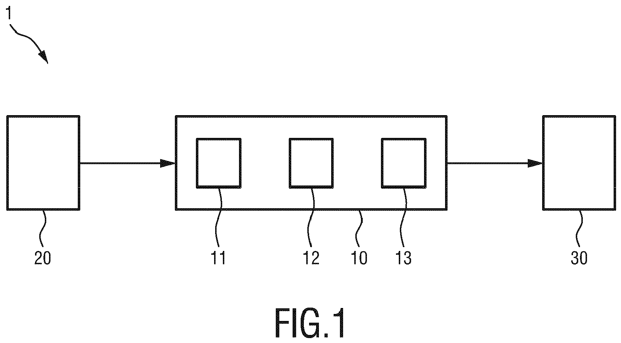

Fig. 1 shows a schematic diagram of an embodiment of a system 1 and a device 10 for generating information on musculoskeletal recovery of a subject according to the present invention. The system 1 comprises the device 10 that is configured to generate information on musculoskeletal recovery of the subject from a motion parameter and/or motion signal of the subject. The system 1 further comprises a sensor 20 configured to measure a motion parameter and/or motion signal related to motion of the subject and a user interface 30 to issue the generated information on musculoskeletal recovery, e.g. to the subject doing the training or another user such as a coach or teacher.

Different kinds of sensors may be used in the system 1. Generally, one sensor may be sufficient, but multiple sensors may be used as well. The sensor 20 may e.g. include one or more of a wearable accelerometer, wearable force sensor, wearable pressure sensor, wearable electromyography sensor, wearable global positioning system sensor, remote camera, radar, speed measurement sensor, and a torque measurement sensor and/or wherein the sensor (20) is an arm- or wrist-worn sensor. Further, a sensor in fitness equipment such as a sensor measuring speed of a treadmill or measuring torque on a seated leg curl machine may be used.

Different kinds of user interface 30 may be used in the system 1. Generally, one user interface may be sufficient, but multiple sensors may be used as well. The user interface 30 may be configured to allow input of subject-related and/or environment-related information, in particular of one or more of a type of surface, a type of shoes of the subject, body height of the subject, weight of the subject, age of the subject, and existing and/or previous injuries of the subject.

The device 10 generally comprises a sensor input 11, a processor 12 and an output 13. The sensor input 11 is configured to receive a motion parameter and/or motion signal related to motion of the subject performing an activity. It may e.g. be data receiving element, e.g. a Bluetooth interface or a cable connection, to receive data from the sensor 20. The processor 12 is configured to determine from the motion parameter and/or motion signal a measure of impact load, determine from the measure of impact load a measure of musculoskeletal damage, and determine from the measure of impact load information on musculoskeletal recovery indicating to which extent the musculoskeletal damage recovers over time. Details of this processing as well as various embodiments will be explained below. The output 13 is configured to output the determined information on musculoskeletal recovery. The output 13 may e.g. be a data transmission element, e.g. a Bluetooth interface or a cable connection, to provide data to the user interface 30.

The device 10 may be implemented in soft- and/or hardware, for instance by a programmed processor or computer. Device 10, sensor 20 and user interface 30 may be integrated as a common device, e.g. a wearable device worn by the subject. Alternatively, they may be implemented as separate entities, e.g. as a wearable sensor 20 worn by the subject that transmits the measured motion parameter and/or motion signal, preferably in a wireless manner (e.g. via Bluetooth), to the device 10, where the measured motion parameter and/or motion signal are evaluated, the result of which is provided to the user interface 30 for informing the subject or another person, such a as coach of the subject.

In a first embodiment, the invention makes use of a body-worn accelerometer as sensor 20. Accelerometers are very common to wear during running. They can be found in a watch, a chest strap, a food pod, and a phone (often worn on the upper arm), etc. Instead of these common locations to wear an accelerometer, the present invention also works with an accelerometer on another place, e.g. in headphones or on shorts. The exact way of how to process the accelerometer signals to get a measure for impact load may depend on the location though.

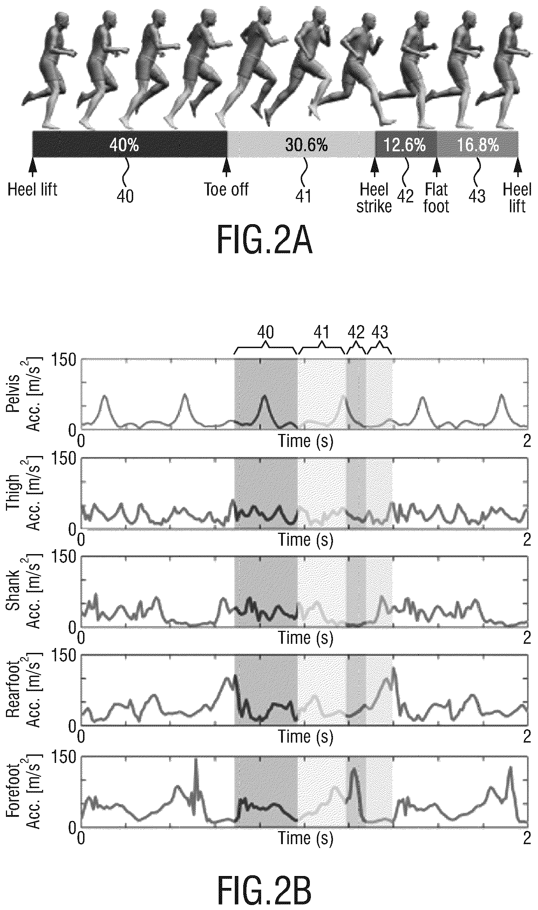

Fig. 2A shows a diagram of the gait cycle phases during running and Fig. 2B shows accelerometer signals (taken from Kwakkel et al. "GNSS Aided In Situ Human Lower Limb Kinematics During Running" ION GNSS 2008) for accelerometers placed on different places on the body during running. The different gait cycle phases (push-off 40, swing 41, heel-strike 42, stance 43) in each strike can be seen, especially here for the pelvis, rear foot and forefoot. For each of those accelerometers the maximum acceleration per strike may be used as a measure for impact load per strike. To get the total impact load for the training, these should be summed for every strike (and times two to get it per step, assuming symmetrical running). As force is given by the acceleration times the mass, it is also possible to include the mass of the subject to get a better estimate for the impact load (e.g. by multiplying the maximum acceleration by the mass and the number of steps). Instead of trying to calculate the impact load from a real force, it is also possible to use surrogate measures, such as the standard deviation of the acceleration per stride.

As for a wrist-worn accelerometer the body damps already part of the shock from the impact on the ground, it is harder (compared to a foot- or hip-worn accelerometer) to derive the force from the impact directly from the accelerometer signal. However, it is known that speed and cadence can be derived from a wrist-worn accelerometer signal. Dividing the speed by the cadence gives the average step length. The impact load per step is related to the step length (a larger step length leads to a higher impact load). Therefore, also wrist-worn accelerometers can be used for the present invention.

Instead of accelerometers, pressure sensors in the insoles of shoes can be used. These give a direct measure for the impact load per step, which should again be summed for all steps during a training to get the total impact load for the training.

All kinds of other (surrogate) measurements for impact load are generally possible. For example, the impact load can be estimated as being proportional to an EMG signal measured with electrodes on the subject's leg. Besides, the impact load can be estimated with non-wearable sensors, e.g. with pressure sensors on a treadmill (underneath the belt, not moving with the belt), or from camera images, e.g. with a camera pointed on the legs when running on a treadmill or with cameras along an athletics track.

The embodiments described above are preferably useful for measurements for running. The present invention can however also be used for other sports, such as fitness. The impact load may then be measured with the fitness equipment. For example, a seated leg curl machine can measure the torque per repetition and the number of repetitions. Multiplying the two measurements gives a measure for impact load. Of course, measuring impact load in fitness can also be done with wearable sensors, instead of with the equipment. The following description will mainly focus on runners and the mechanical impact load that results from running trainings.

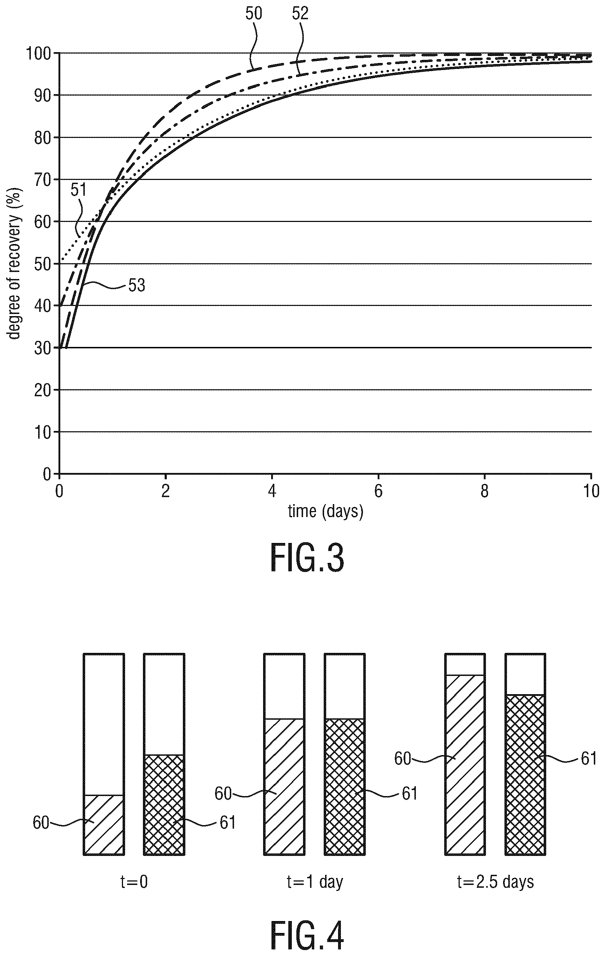

Fig. 3 shows a diagram illustrating an example of cardiovascular recovery 50, recovery from mechanical impact 51 and two combined recoveries 52, 53 (obtained by different measurements, in particular the average 52 and the minimum 53) for a training that ended on t=0. In this example, the cardiovascular system was challenged quite a lot during the training, thereby resulting in a cardiovascular condition of only 30% of a fully recovered condition. The mechanical impact load of the training resulted in a musculoskeletal condition of 50% of a fully recovered condition.

In this case, the cardiovascular system recovers according to formula:

$$\text{degree of cardiovascular recovery (\%)} = 100\% - C1 * EXP(-t/\ TAU1),$$

wherein t is the time, C1 is the cardiovascular condition loss at the end of the training (t=0), which is e.g. equal to 70%, and TAU1 is a time constant for cardiovascular recovery, which is e.g. equal to 1.25 days.

Furthermore, in this case, the musculoskeletal recovery is given by:

$$\text{degree of musculoskeletal recovery (\%)} = 100\% - C2 * EXP(-t/\ TAU2),$$

wherein C2 is the musculoskeletal condition loss at the end of the training (t=0), which is e.g. equal to 50%, and TAU2 is a time constant for musculoskeletal recovery, which is e.g. equal to 2.5 days.

Since in this case the cardiovascular system recovers faster than the musculoskeletal system, after 2.5 days, the cardiovascular system is already 90.5% of fully recovered while the musculoskeletal system is only 81.6 of fully recovered (meaning the optimal situation including supercompensation).

Feedback to the user can be given in different forms. One embodiment is illustrated in Fig. 4, according to which the values of the recovery for the cardiovascular system 60 and the musculoskeletal system 61 are visually indicated separately over time. In particular, feedback may be given right after the training, after 1 day, and after 2.5 days.

Instead of showing bars, the feedback may also just be given as numbers, e.g. percentage or a number between 0 and 10, or the like. Instead of increasing over time, the opposite can be shown; a value decreasing towards zero, where zero means fully recovered (i.e. zero damage left over from the training). Another option is to give the optimal time or time window to start a new training.

Instead of, or in addition to showing the recovery for the cardiovascular system and the musculoskeletal system separately, the two recoveries may be combined into one recovery indication. Two methods to do this have been illustrated above with reference to Fig. 3. The first method takes the average (curve 52 in Fig. 3) of the cardiovascular and the musculoskeletal recovery, whereas the second method takes the minimum (curve 53 in Fig. 3). Other mathematical operations to combine the two are possible as well, for example giving one of the two more weight than the other.

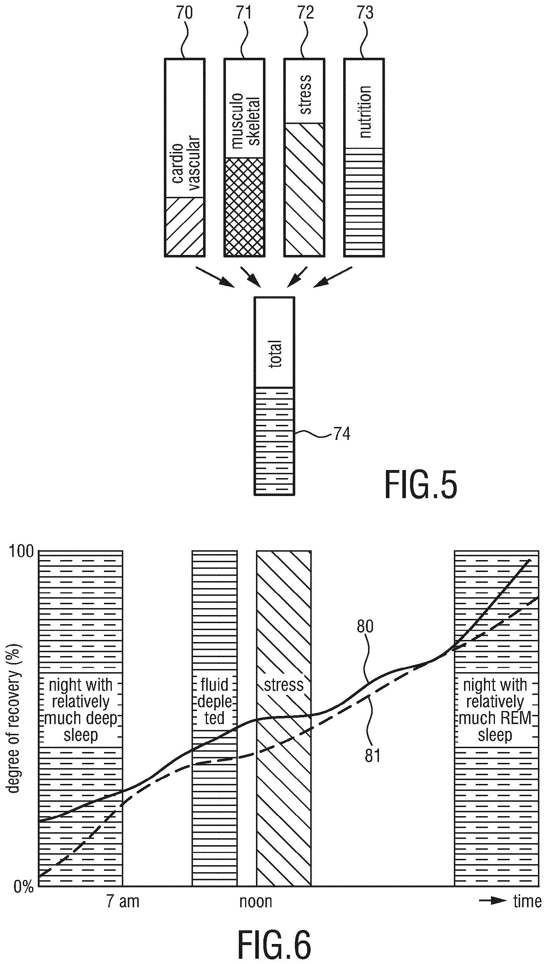

In principle, even more pillars that contribute to a person's condition and/or recovery can be shown and/or combined. The total may be seen as the trainability of a person, as exemplarily shown in Fig. 5 showing pillars for cardiovascular 70, musculoskeletal 71, stress 72, and nutrition73 and all combined into one bar 74 giving the trainability of a person. Next to the shown cardiovascular, musculoskeletal, stress, and nutrition pillars, one or more pillars relating to illness/symptoms/medication or relating to fluid level may be added as well.

In the example illustrated above with respect to Fig 3, the start values (at t=0) for the cardiovascular and musculoskeletal condition were determined from TRIMP (training impulse, which is a measure of workload of a training based on heart rate zones or rate of perceived exertion and their duration) and impact load of the training respectively (and possibly additionally left over from earlier training(s) if the last training was started before the person was fully recovered). In other words: for the equations illustrated above, the TRIMP determined C1 and the impact load determined C2. TAU1 and TAU2 were constants. They could be default values, which are taken the same for any subject (wherein TAU1 is different from TAU2).

In a more advanced embodiment of the present invention, TAU1 and TAU2 may be personalized: for a person in a very good condition and eating healthy, TAU1 and TAU2 are smaller than for a person that has a bad condition that only eats junk food.

In an even more advanced embodiment of the invention, TAU2 could depend on the measured motion or impact load. For example, if the impact load measurement was indicative for heel strike, TAU2 may be taken larger than when the measurement revealed forefoot strike, because the joints impacted recover slower. Apart from influencing the speed of recovery (TAU2), the way of running (e.g. heel strike vs midfoot strike vs. forefoot strike) may also be taken into account in the calculation of the musculoskeletal condition at t=0 (thus influencing C2 for the equation of the example).

Instead of an exponential equation, like those illustrated above, other equations may be used, like the degree of recovery (%) = 100(%) - c/(a*t+b), with a, b, and c being constants.

Instead of using simple equations that continue to be valid from the end of the training until the beginning of the next training, more complex relations can be used, which depend on conditions that the user is experiencing, like stress, sleep quality, fluid level, etc. For example, periods with stress or a low fluid level might slow down the recovery and the normal recovery rate might pick up again when the stress has gone and the fluid level has restored. Stress might have more impact on the speed of cardiovascular recovery, whereas the fluid level might have more impact on the speed of musculoskeletal recovery. Massage or heat (e.g. measured with a skin temperature sensor) will speed up the musculoskeletal recovery, while it has little influence on the cardiovascular recovery. Also sleep might impact the speed of cardiovascular recovery differently than the speed of musculoskeletal recovery; REM (rapid eye movement) sleep is important for mental recovery (which on its turn is important for cardiovascular recovery), whereas deep sleep is important for musculoskeletal recovery. A body-worn (e.g. wrist-worn) device could measure the sleep stages, and thus the amount of REM sleep and the amount of deep sleep. From this information, the influence on the cardiovascular and musculoskeletal recovery respectively can be determined. An example of such complex relations to use is given in Fig. 6.

Thus, the speed of recovery may follows a default formula (e.g. using parameters that are constant in time and independent of the user, but instead a formula is used for all users of the system), follow a formula with personalized parameters, follow a formula dependent on the motion during the training that caused the damage, or has a more fluctuating behaviour, which also takes into account the situation, like the amount of deep sleep or total sleep time, massages or skin temperature.

Fig. 6 shows a diagram illustrating the degree of recovery for the cardiovascular system 80 and the degree of recovery for the musculoskeletal system 81 over 24 hours. The first night has relatively much deep sleep (and relatively little REM sleep), which results in a fast recovery of the musculoskeletal system (and a slower recovery for the cardiovascular system), whereas the second night has relatively much REM sleep (and relatively little deep sleep), resulting in fast recovery of the cardiovascular system (and slower for the musculoskeletal system). Fluid depletion in the morning slows down the musculoskeletal recovery, while it has little effect on the cardiovascular recovery. On the other hand, stress, just after noon, slows down the cardiovascular recovery, while it has little effect on the musculoskeletal recovery.

It shall be noted that in many of the graphs and explanations the recovery of the musculoskeletal system and the cardiovascular system are both shown. The present invention, however, is separate and independent of the cardiovascular recovery, but is mainly directed to the musculoskeletal recovery that can be determined separately and alone; determining the cardiovascular recovery is optional according to the present invention.

The cardiovascular recovery is mainly shown as a comparison to commonly known systems that determine and output the cardiovascular recovery. The musculoskeletal recovery can be different (and most of the time is different) from the cardiovascular recovery, both regarding speed of recovery, as well as to which extent there is loss of condition initially, due to the training, directly at the end of a training (considering e.g. an athlete doing a training that consists of 15 x 100m sprint, with much rest in between; his heart rate will not reach high zones (and therefore the TRIMP is low and the cardiovascular system does not suffer much), while his muscles, joints and tendons suffer a lot from the training).

The musculoskeletal damage may be determined for running episodes; the impact load is coming from the foot-ground impact (according to Newton's third law: When a first body exerts a force on a second body, the second body simultaneously exerts a force equal in magnitude and opposite in direction on the first body).

It is known that running on damped surfaces (like on sand in the woods) gives less of a burden to the musculoskeletal system then running on e.g. asphalt. Some of the above-described embodiments directly incorporate that because the peak force that they measure per stride is lower (e.g. with pressure insoles in the shoes or with an accelerometer attached to the foot or hip). However, other embodiments to determine the impact load, like when EMG is used (and to a certain extent also for a wrist-worn accelerometer), may need to take this into account as an additional factor. These embodiments may get the information that the surface was damped either from user input or from measurements from other sensors. Moreover, it is possible to derive the information from the sensor itself: steps are more irregular on damped surfaces (as e.g. easily clear for a person running in the woods or on the beach), which will reflect in the signals (e.g. EMG or wrist-worn accelerometer) as varying from step to step. From this irregularity, a damped surface may be detected. Another way to detect a damped surface may use the sound of the steps, measured with a microphone. When a damped surface has been detected, this might be accounted for in the derived impact load, e.g. by multiplying the value by a factor, such as 0.8.

Similarly, the total impact per step is smaller when a person is landing on his forefoot compared to when he is landing on his heels. When the impact load is measured e.g. from an accelerometer on the hip, this may directly be taken into account in the determination of the impact load. However, some other embodiments to determine impact load (e.g. with an accelerometer on the wrist) might not directly measure this. The measured measure for impact load may then be scaled with a factor to account for forefoot landing (e.g. multiply by 0.8) vs. heel strike (e.g. multiply by 1.2).

The information on whether the person is landing on his heels or on his forefoot may be taken from other sensors (like pressure sensors on insoles in the shoes), from user input, or from the cadence (i.e. the step rate). The reasoning behind the latter is that it is more natural to land on forefoot when the cadence is high; e.g. for a cadence of 180 or higher it might be assumed that the person is landing on his forefoot. Cadence is an easy parameter to measure; the sensor that is used to derive the impact load can at the same time be used to measure the cadence. The speed of recovery (reflected as TAU2 in the formula illustrated above) might also differ for forefoot strike vs. heel strike, especially because different body parts are damaged to different extent.

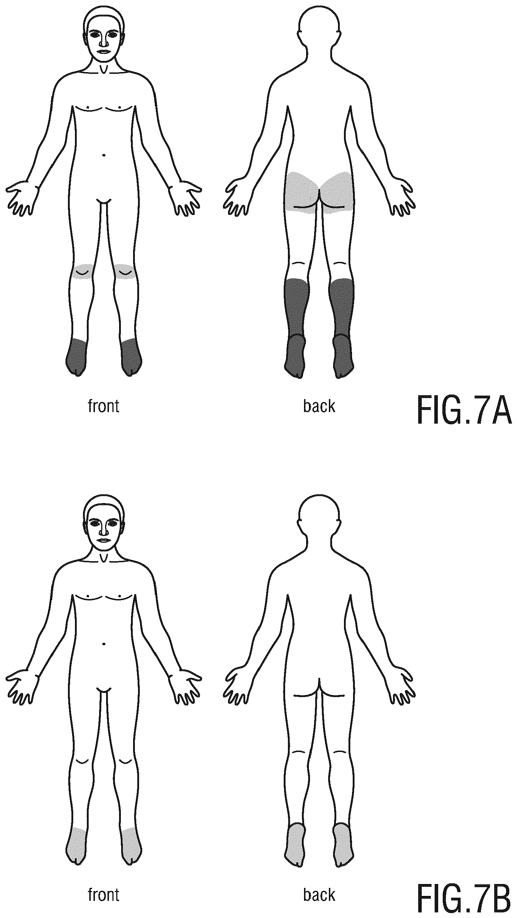

In the above, the musculoskeletal system was treated as a whole. However, it is possible to discriminate between different body parts. For example, for forefoot landing, feet and calves suffer most, while for heel strike the knees get larger shocks. Further, not all body parts will recover at the same speed, because some body parts are less perfused than others. This can be depicted in pictures that are shown to the user. For instance, pictures as shown Figs. 7A and 7B may be depicted to the user.

Figs. 7A and 7B illustrate a kind of map showing which body parts are affected and to which extent. Fig. 7A shows a status right after running with mainly forefoot strike: feet and calves suffered most and to a lesser extent the knees and buttocks; the rest of the legs even less. Fig. 7B shows a status about twelve hours later: After the training most of the legs have recovered and only the calves still have some damage, but the damage is less than the damage in the feet, because the latter were less perfused and therefore recovered slower.

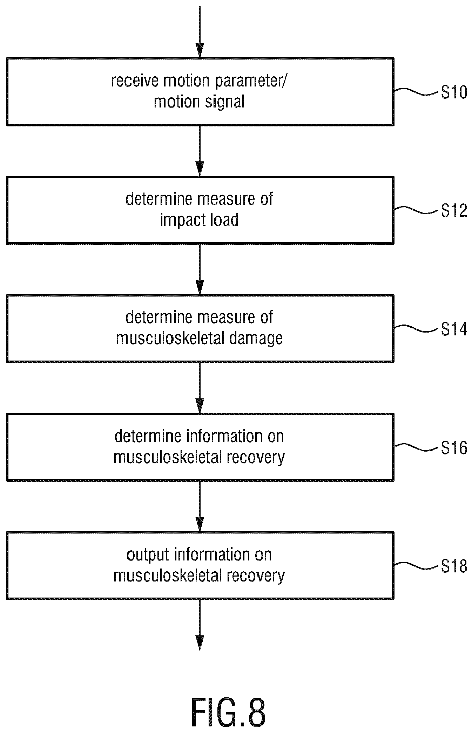

Fig. 8 shows a flowchart illustrating an embodiment of a method for generating information on musculoskeletal recovery of a subject according to the present invention. In an initial step S10 a motion parameter and/or motion signal related to motion of the subject is received. From the motion parameter and/or motion signal a measure of impact load is determined in step S12. From the measure of impact load a measure of musculoskeletal damage is determined in S14. Then, in step S16, information on musculoskeletal recovery indicating to which extent the musculoskeletal damage recovers over time is determined from the measure of impact load. Finally, in step S18, the determined information on musculoskeletal recovery is output.

The present invention can support runners, but other sportsmen exercising another sport or a combination of sports. Further, it may be valuable for various kinds of patients, such as pre- and post-operative patients.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for generating information on musculoskeletal recovery of a subject, said device comprising

   - a sensor input (11) configured to receive a motion parameter and/or motion signal related to motion of the subject performing an activity;
   - a processor (12) configured to

      determine from the motion parameter and/or motion signal a measure of impact load,
      determine from the measure of impact load a measure of musculoskeletal damage, and
      determine from the measure of impact load information on musculoskeletal recovery indicating to which extent the musculoskeletal damage recovers over time; and

   - an output (13) configured to output the determined information on musculoskeletal recovery.

**2.** Device according to claim 1,
wherein the processor (12) is configured to determine the measure of impact load from one or more of the maximum force of a step times the number of steps, the maximum force of a step times the cadence times the duration at this cadence, the speed times the duration of the speed, average magnitude of acceleration times its duration, the peak magnitude of acceleration times its duration, integral of EMG signal over time, maximum torque or force per repetition times the number of repetitions, vertical oscillation times the number of steps, and step length times the number of steps.

**3.** Device according to any one of the preceding claims,
wherein the processor (12) is configured to determine the measure of musculoskeletal damage based on a predetermined relationship, in particular a linear relationship, with the determined measure of impact load.

**4.** Device according to any one of the preceding claims,
wherein the processor (12) is configured to determine the information on musculoskeletal recovery from the measure of impact load information by use of musculoskeletal condition loss at a known moment in time and a time constant for musculoskeletal recovery.

**5.** Device according to any one of the preceding claims,
wherein the processor (12) is configured to determine the information on musculoskeletal recovery based on one of a default formula, a formula with personalized parameters, a formula dependent on the motion of the subject, or a formula having a fluctuating behaviour.

**6.** Device according to any one of the preceding claims,
wherein the processor (12) is configured to determine the information on musculoskeletal recovery including one or more of a recovery time indicating the optimal time the subject needs to wait before a new activity should be started, a damage number that decreases over time when there is no new activity, and a recovery number that increases over time when there is no new activity.

**7.** Device according to any one of the preceding claims,
wherein the processor (12) is configured to determine, in addition to information on musculoskeletal recovery, additional information on cardiovascular recovery and/or on another recovery-related aspect, and
wherein the output (13) is configured to output the determined additional information separate from the information on musculoskeletal recovery or as combined information on recovery determined from the information on musculoskeletal recovery and the additional information, wherein the another recovery-related aspect is one or more of nutrition, fluid level, mental energy, cardiovascular stamina, illness, symptoms, and medication.

**8.** Device according to any one of the preceding claims,
wherein the processor (12) is configured to determine the measure of musculoskeletal damage from the measure of impact load at the latest activity of the subject and optionally in addition from one or more earlier activities of the subject.

**9.** Device according to any one of the preceding claims,
wherein the processor (12) is configured to take into account, in determining the measure of impact load, the hardness of the surface on which the subject performs the activity and/or a foot strike pattern.

**10.** Device according to any one of the preceding claims,
wherein the processor (12) is configured to determine the information on musculoskeletal recovery separately for one or more different body parts.

**11.** System for generating information on musculoskeletal recovery of a subject, said system comprising

- a sensor (20) configured to measure a motion parameter and/or motion signal related to motion of the subject;
- a device (10) configured to generate from the motion parameter and/or motion signal information on musculoskeletal recovery of the subject; and
- a user interface (30) to issue the generated information on musculoskeletal recovery.

**12.** System according to claim 11,
wherein the sensor (20) includes one or more of a wearable accelerometer, wearable force sensor, wearable pressure sensor, wearable electromyography sensor, wearable global positioning system sensor, remote camera, radar, speed measurement sensor, and a torque measurement sensor and/or wherein the sensor (20) is an arm- or wrist-worn sensor.

13. System according to claim 11 or 12,
   wherein the user interface (30) is configured to allow input of subject-related and/or environment-related information, in particular of one or more of a type of surface, a type of shoes of the subject, body height of the subject, weight of the subject, age of the subject, and existing and/or previous injuries of the subject.

14. Method for generating information on musculoskeletal recovery of a subject, said method comprising

   - receiving a motion parameter and/or motion signal related to motion of the subj ect;
   - determining from the motion parameter and/or motion signal a measure of impact load;
   - determining from the measure of impact load a measure of musculoskeletal damage;
   - determining from the measure of impact load information on musculoskeletal recovery indicating to which extent the musculoskeletal damage recovers over time; and
   - outputting the determined information on musculoskeletal recovery.

15. Computer program comprising program code means for causing a computer to carry out the steps of the method as claimed in claim 14 when said computer program is carried out on the computer.

FIG.1

FIG.2A

FIG.2B

FIG.3

FIG.4

70    71    72    73

cardio vascular

musculo skeletal

stress

nutrition

total

74

# FIG.5

degree of recovery (%)

100

night with relatively much deep sleep

fluid deple- ted

stress

80

81

night with relatively much REM sleep

0%

7 am                    noon                                              time

# FIG.6

front       back      FIG.7A

front       back      FIG.7B

```
         │
         ▼
┌─────────────────────────────┐
│   receive motion parameter/ │ ⌒ S10
│       motion signal         │
└─────────────────────────────┘
         │
         ▼
┌─────────────────────────────┐
│     determine measure of    │ ⌒ S12
│        impact load          │
└─────────────────────────────┘
         │
         ▼
┌─────────────────────────────┐
│     determine measure of    │ ⌒ S14
│    musculoskeletal damage    │
└─────────────────────────────┘
         │
         ▼
┌─────────────────────────────┐
│    determine information on  │ ⌒ S16
│   musculoskeletal recovery   │
└─────────────────────────────┘
         │
         ▼
┌─────────────────────────────┐
│     output information on    │ ⌒ S18
│   musculoskeletal recovery   │
└─────────────────────────────┘
         │
         ▼
```

# FIG.8

Europäisches
Patentamt

European
Patent Office

Office européen
des brevets

**EUROPEAN SEARCH REPORT**

Application Number

EP 20 16 7671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/183412 A1 (HUIJBREGTS LAURENTIA JOHANNA [NL] ET AL) 20 June 2019 (2019-06-20) * paragraphs [0079] - [0097] * ----- | 1-15 | INV. A61B5/11 |
| X | US 2019/184232 A1 (PULKKINEN AKI [FI] ET AL) 20 June 2019 (2019-06-20) * paragraphs [0060], [0100] - [0111] * ----- | 1-15 | |
| X | US 2019/328284 A1 (FINCH MARK [NZ] ET AL) 31 October 2019 (2019-10-31) * paragraphs [0007] - [0009], [0137] - [0193] * ----- | 1-15 | |
| X | US 2016/058378 A1 (WISBEY BEN [AU] ET AL) 3 March 2016 (2016-03-03) * paragraphs [0045] - [0047], [0076], [0077], [0114] - [0164] * ----- | 1-15 | |

TECHNICAL FIELDS
SEARCHED     (IPC)

A61B
G16H
A63B

The present search report has been drawn up for all claims

| Place of search | Date of completion of the search | Examiner |
|---|---|---|
| Berlin | 8 September 2020 | Trachterna, Morten |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 16 7671

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2020

| Patent document cited in search report | | Publication date | Patent family member(s) | | Publication date |
|---|---|---|---|---|---|
| US 2019183412 | A1 | 20-06-2019 | AU | 2017309761 A1 | 28-03-2019 |
| | | | CN | 109561837 A | 02-04-2019 |
| | | | EP | 3496600 A1 | 19-06-2019 |
| | | | JP | 2019524287 A | 05-09-2019 |
| | | | US | 2019183412 A1 | 20-06-2019 |
| | | | WO | 2018029171 A1 | 15-02-2018 |
| US 2019184232 | A1 | 20-06-2019 | NONE | | |
| US 2019328284 | A1 | 31-10-2019 | AU | 2015237956 A1 | 06-10-2016 |
| | | | AU | 2020202268 A1 | 23-04-2020 |
| | | | GB | 2539590 A | 21-12-2016 |
| | | | US | 2018020950 A1 | 25-01-2018 |
| | | | US | 2019328284 A1 | 31-10-2019 |
| | | | WO | 2015145273 A1 | 01-10-2015 |
| US 2016058378 | A1 | 03-03-2016 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7192401 B
- US 2016220866 A1

**Non-patent literature cited in the description**

- **KWAKKEL et al.** GNSS Aided In Situ Human Lower Limb Kinematics During Running. *ION GNSS,* 2008